# EUROPÄISCHE PATENTSCHRIFT

(11) **EP 3 713 604 B1**
(45) Veröffentlichungstag und Bekanntmachung des Hinweises auf die Patenterteilung: **07.06.2023**
(21) Anmeldenummer: 18815116.1
(22) Anmeldetag: 23.11.2018
(51) Int. Cl.: A61K 31/216, A61K 45/06, A61K 31/25, A61P 25/28

(54) **VERBINDUNG ZUR ANWENDUNG BEI DER STEIGERUNG VON MENTALER LEISTUNGSFÄHIGKEIT**
COMPOUND FOR USE IN THE INCREASING OF MENTAL CAPABILITY
COMPOSÉ À UTILISER LORS DE L'AUGMENTATION DE PERFORMANCES INTELLECTUELLES

(30) Priorität: 24.11.2017 DE 102017127865
(43) Veröffentlichungstag der Anmeldung: 30.09.2020
(73) Patentinhaber: Leibniz- Institut für Neurobiologie (LIN) Stiftung des öffentlichen Rechts, 39118 Magdeburg (DE); Leibniz-Institut für Pflanzenbiochemie (IPB) Stiftung des öffentlichen Rechts, 06120 Halle (Saale) (DE); Deutsches Zentrum für Neurodegenerative Erkrankungen e.V. (DZNE), 53127 Bonn (DE)
(72) Erfinder: MICHELS, Birgit, 39104 Magdeburg (DE); GERBER, Bertram, 39114 Magdeburg (DE); WESSJOHANN, Ludger, 06120 Halle (Saale) (DE); FRANKE, Katrin, 06120 Halle (Saale) (DE); SIGRIST, Stephan, 12163 Berlin (DE); BHUKEL, Anuradha, Ashburn, Virginia (VA) 20147 (US); LUSHCHAK, Oleh, Ivano-Frankivsk 76492 (UA); ZWAKA, Hanna, 10829 Berlin (DE); BARTELS, Ruth, 10629 Berlin (DE); DITYATEV, Alexander, 39175 Wahlitz (DE); SONG, Inseon, 39114 Magdeburg (DE); FENDT, Markus, 39104 Mageburg (DE); LESSMANN, Volkmar, 39108 Magdeburg (DE); ENDRES, Thomas, 39104 Magdeburg (DE); KÄHNE, Thilo, 39130 Magdeburg (DE)
(74) Vertreter: Dr. Klemens Schubert Patentanwalt
(86) Internationale Anmeldenummer: PCT/EP2018/082420
(87) Internationale Veröffentlichungsnummer: WO 2019/101952

(56) Entgegenhaltungen:
- RAZIEH HOSSEINI ET AL: "Discovery of neurotrophic agents based on hydroxycinnamic acid scaffold", CHEMICAL BIOLOGY & DRUG DESIGN., Bd. 88, Nr. 6, 9. September 2016 (2016-09-09), Seiten 926-937, XP055558196, GB ISSN: 1747-0277, DOI: 10.1111/cbdd.12829
- MOOSAVI FATEMEH ET AL: "Derivatives of caffeic acid, a natural antioxidant, as the basis for the discovery of novel nonpeptidic neurotrophic agents", BIOORGANIC & MEDICINAL CHEMISTRY, PERGAMON, GB, Bd. 25, Nr. 12, 27. April 2017 (2017-04-27), Seiten 3235-3246, XP085030764, ISSN: 0968-0896, DOI: 10.1016/J.BMC.2017.04.026
- YONG-JUN CHOI ET AL: "Cytoprotective Effects of Docosyl Cafferate against tBHP-Induced Oxidative Stress in SH-SY5Y Human Neuroblastoma Cells", BIOMOLECULES & THERAPEUTICS, Bd. 19, Nr. 2, 30. April 2011 (2011-04-30) , Seiten 195-200, XP055558313, KR ISSN: 1976-9148, DOI: 10.4062/biomolther.2011.19.2.195
- TAGUCHI RIHO ET AL: "Structure-activity relations of rosmarinic acid derivatives for the amyloid [beta] aggregation inhibition and antioxidant properties", EUROPEAN JOURNAL OF MEDICINAL CHEMISTRY, EDITIONS SCIENTIFIQUE ELSEVIER, PARIS, FR, Bd. 138, 18. Juli 2017 (2017-07-18), Seiten 1066-1075, XP085163750, ISSN: 0223-5234, DOI: 10.1016/J.EJMECH.2017.07.026
- Birgit Michels ET AL: "Memory enhancement by ferulic acid ester across species", SCIENCE ADVANCES, 24. Oktober 2018 (2018-10-24), XP055557976, Gefunden im Internet: URL:http://advances.sciencemag.org/content /4/10/eaat6994.full.pdf [gefunden am 2019-02-18]
- AGOSTINO PALMERI ET AL: "Salidroside, a Bioactive Compound of Rhodiola Rosea, Ameliorates Memory and Emotional Behavior in Adult Mice", JOURNAL OF ALZHEIMER'S DISEASE, Bd. 52, Nr. 1, 26. April 2016 (2016-04-26) , Seiten 65-75, XP055558659, NL ISSN: 1387-2877, DOI: 10.3233/JAD-151159
- JOSHI G ET AL: "In vivo protection of synaptosomes by ferulic acid ethyl ester (FAEE) from oxidative stress mediated by 2,2-azobis(2-amidino-propane)dihydrochlori de (AAPH) or Fe^2^+/H"2O"2: Insight into mechanisms of neuroprotection and relevance to oxidative stress-related neurodegenerative disorders", NEUROCHEMISTRY INTERNATIONAL, ELSEVIER, AMSTERDAM, NL, Bd. 48, Nr. 4, 1. März 2006 (2006-03-01), Seiten 318-327, XP027957858, ISSN: 0197-0186 [gefunden am 2006-03-01]
- NABAVI SEYED FAZEL ET AL: "Rhodiola rosea L. and Alzheimer's Disease: From Farm to Pharmacy", PHYTOTHERAPY RESEARCH, JOHN WILEY & SONS LTD, GB, Bd. 30, Nr. 4, 1. April 2016 (2016-04-01), Seiten 532-539, XP002784850, ISSN: 1099-1573, DOI: 10.1002/PTR.5569

## Beschreibung

Das technische Gebiet, auf das sich die Erfindung bezieht, betrifft eine Verbindung zur Anwendung bei der Steigerung von mentaler Leistungsfähigkeit sowie eine diese Verbindung umfassende pharmazeutische Zusammensetzung.

Hauptrisikofaktor für Demenz ist das hohe Lebensalter. Aufgrund der demographischen Entwicklung der Gesellschaft treten Demenzerkrankungen wie z.B. die Alzheimer-Krankheit deshalb immer häufiger auf. Parallel dazu spielt in einer leistungsorientierten Gesellschaft die geistige Fitness eine zunehmend wichtige Rolle. Laut einer Online-Umfrage des Wissenschaftsjournals Nature griffen im Jahre 2008 von 1400 gesunden Wissenschaftlern 20 % zu Medikamenten, um ihre Aufmerksamkeit, Konzentration oder Gedächtnisleistung zu steigern. Dieser gesellschaftliche Bedarf, sowohl im Kontext der Demenzerkrankungen wie auch im Kontext der Leistungssteigerung, führte unter anderem zur Entwicklung von Präparaten, die Substanzen, synthetischen wie natürlichen Ursprungs, zum Zweck der Steigerung der Gedächtnisleistung enthalten. Oft handelt es sich um pflanzliche Präparate.

Die folgenden Verbindungen oder Pflanzen sind in Bezug auf die Lern- und Gedächtnisleistung bekannt und werden in diesem Zusammenhang vermarktet: Racetame (Piracetam und Analoga), Amphetamine, Methylphenidat, Sabeluzole, Exifone, Leteprinim, AChE-Hemmer (z.B. Tacrin, Donepezil, Rivastigmin, Galantamin, Huperzine A (*Huperzia serrata* (Thunb.) *Trevis*), Omega-3-Fettsäuren (z.B. Fischöl, *Salmo salar* L.), Curcurmin (*Curcuma longa* L.), Vincamin (*Vinca minor L.*), *Ginkgo biloba* L., *Panax ginseng* C.A.Mey, *Bacopa monnieri* (L.) Wettst., *Evolvulus alsinoides* L., *Lepidium meyenii* Walp., *Eleutherococcus senticosus* (Rupr. & Maxim.) Maxim, *Erythroxylum coca* Lam, *Zingiber officinale* Roscoe, *Hypericum perforatum* L. und *Rhodiola rosea* L.

Die genauen Auswirkungen dieser Substanzen, insbesondere vieler Pflanzenprodukte, auf das Gedächtnis sind gewöhnlich nicht bekannt. Überwiegend setzen sich die auf dem Markt befindlichen Produkte aus Pflanzenextrakten zusammen, welche auf einzelne Inhaltsstoffe der verwendeten Pflanzenteile normiert wurden (Referenzsubstanz), für die jedoch keinerlei kausaler Zusammenhang zur Wirkung bestehen muss. Daher stimmt die bioaktive Substanz innerhalb des Pflanzenteils oft nicht mit der Referenzsubstanz überein, denn die Referenzsubstanz wird gewöhnlich nach analytischen Gesichtspunkten ausgewählt, z.B. als typische Komponente der besagten Pflanze, um Verwechslungen mit ähnlichen Pflanzen zu vermeiden. In solchen Fällen ist mit stark schwankenden Konzentrationen des bioaktiven Wirkstoffes innerhalb verschiedener Produktchargen zu rechnen, welche Schwankungen der Effektivität des Produkts und Fehldosierungen zur Folge haben. Dieses grundsätzliche Problem in der Anwendung pflanzenbasierter Präparate kann nur durch die Verwendung konkreter Wirkstoffe mit kausal nachgewiesener Aktivität gelöst werden. Razieh Hosseini et al., Chemical Biology & Drug Design, Bd. 88, 2016, S. 926-937, offenbart einen protektiven Effekt von Kaffeesäurehexylester, -octylester, -decylester und -dodecylester auf Nervenzellen.

Aufgabe der vorliegenden Erfindung ist es daher diese Nachteile des Standes der Technik zu überwinden und eine Verbindung bereitzustellen, die gegenüber den bekannten Substanzen die mentale Leistungsfähigkeit spezifisch und signifikant steigern kann.

Die Aufgabe wird durch die Bereitstellung einer Verbindung zur Anwendung bei der Steigerung von mentaler Leistungsfähigkeit gemäß den Merkmalen des Hauptanspruchs gelöst. Vorteilhafte Ausgestaltungen der erfindungsgemäßen Verbindung sind in den abhängigen Unteransprüchen gekennzeichnet.

Gegenstand der vorliegenden Erfindung ist eine Verbindung gemäß der allgemeinen Formel I 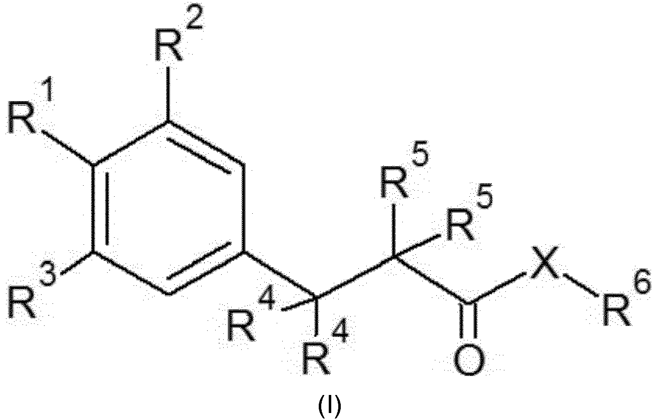 wobei
R1 Hydroxy ist,
R2 ausgewählt ist aus der Gruppe bestehend aus H, Methoxy, Ethoxy, n-Propoxy und i-Propoxy;
und R3 ausgewählt ist aus der Gruppe bestehend aus H, Methoxy, Ethoxy, n-Propoxy und i-Propoxy;
R⁴ und R⁵ jeweils unabhängig voneinander H sind oder jeweils ein R⁴ und ein R⁵ zusammen eine C-C-Bindung bilden, derart, dass zwischen den einander benachbarten C-Atomen, an welchen sich R⁴ und R⁵ befinden, eine Einzel-, Doppel- oder Dreifachbindung vorliegt;
R⁶ ausgewählt ist aus der Gruppe bestehend aus unverzweigten oder verzweigten C₆ bis C₆₀-Alkyl, unverzweigten oder verzweigten C₆ bis C₆₀-Alkenyl, C₆ bis C₆₀-Hydroxypolyethylenglykol, C₆ bis C₆₀-Methoxypolyethylenglykol und C₅ bis C₁₂₀-Prenyl; und
X ausgewählt ist aus der Gruppe bestehend aus O, S, N-H, N-Methyl, N-Ethyl, N-n-Propyl, N-i-Propyl und N-R⁶;
deren Stereoisomere, Enantiomere oder Diastereomere, deren physiologisch annehmbaren Salze sowie Mischungen der genannten Verbindungen zur therapeutischen

Anwendung bei der Steigerung von mentaler Leistungsfähigkeit. Ein weiterer Gegenstand der vorliegenden Erfindung ist die nicht-therapeutisch Anwendung einer Verbindung der Formel I gemäß der obigen Definition bei der Steigerung von mentaler Leistungsfähigkeit.

Insbesondere bevorzugt ist eine Verbindung zur Anwendung bei der Steigerung von mentaler Leistungsfähigkeit, wobei R¹ Hydroxy und R² Methoxy ist. Weiterhin bevorzugt ist eine Verbindung zur Anwendung bei der Steigerung von mentaler Leistungsfähigkeit, wobei R³ H ist.

Bevorzugt ist außerdem eine Verbindung zur Anwendung bei der Steigerung von mentaler Leistungsfähigkeit, wobei die durch R⁴ und R⁵ gebildete C-C-Bindung eine Doppelbindung ist.

Insbesondere bevorzugt ist eine Verbindung zur Anwendung bei der Steigerung von mentaler Leistungsfähigkeit, wobei die Doppelbindung eine E-Doppelbindung ist.

Besonders bevorzugt ist eine Verbindung zur Anwendung bei der Steigerung von mentaler Leistungsfähigkeit, wobei X O ist.

Außerdem ist eine Verbindung zur Anwendung bei der Steigerung von mentaler Leistungsfähigkeit bevorzugt, wobei R⁶ ein C₈ bis C₂₈-Alkyl, bevorzugt ein C₁₂ bis C₂₄-Alkyl und besonders bevorzugt ein C₈-Alkyl, ein C₁₂-Alkyl, ein C₁₆-Alkyl oder ein C₂₀-Alkyl ist.

Weiterhin ist eine Verbindung zur Anwendung bei der Steigerung von mentaler Leistungsfähigkeit bevorzugt, wobei die Verbindung ausgewählt ist aus der Gruppe bestehend aus Icosyl-(2*E*)-3-(4-hydroxy-3-methoxyphenyl)-prop-2-enoat, Octyl-(2*E*)-3-(4-hydroxy-3-methoxyphenyl)-prop-2-enoat, Hexadecyl-(2*E*)-3-(4-hydroxy-3-methoxyphenyl)-prop-2-enoat, Icosyl-3-(4-hydroxy-3-methoxyphenyl)-propanoat, Dodecyl-(2*E*)-3-(4-hydroxy-3-methoxyphenyl)-prop-2-enoat und Icosyl-(2*E*)-3-(4-hydroxyphenyl)-prop-2-enoat.

Besonders bevorzugt ist eine Verbindung zur therapeutischen Anwendung bei der Steigerung von mentaler Leistungsfähigkeit, wobei die mentale Leistungsfähigkeit die Gedächtnisleistung und die Lernleistung umfasst und mit einer Demenzerkrankung assoziiert ist.

Außerdem bevorzugt ist eine pharmazeutische Zusammensetzung zur Anwendung bei der Steigerung von mentaler Leistungsfähigkeit, umfassend mindestens eine erfindungsgemäße Verbindung sowie die physiologisch annehmbaren Salze und Stereoisomere, Enantiomere, Diastereomere oder Mischungen daraus und pharmazeutisch annehmbare Hilfsstoffe und Zusatzstoffe. Im Sinne der vorliegenden Erfindung kann die pharmazeutische Zusammensetzung auch Kombinationen mehrerer der erfindungsgemäßen Verbindungen umfassen.

Besonders bevorzugt ist eine pharmazeutische Zusammensetzung zur Anwendung bei der Steigerung von mentaler Leistungsfähigkeit in Kombination mit weiteren Wirkstoffen, die ausgewählt sind aus der Gruppe bestehend aus Nahrungsergänzungsmitteln, synergistischen Enhancern, additiven Enhancern, Verbindungen zur Verbesserung von Aufnahme und Transport und Mitteln zur Verminderung der Spaltung oder Metabolisierung sowie deren Mischungen.

Insbesondere bevorzugt ist eine pharmazeutische Zusammensetzung zur Anwendung bei der Steigerung von mentaler Leistungsfähigkeit, wobei die pharmazeutische Zusammensetzung zur oralen, intranasalen oder subkutanen Verabreichung hergerichtet ist.

Die mentale Leistungsfähigkeit umfasst im Sinne der vorliegenden Erfindung die Gedächtnisleistung sowie die Lernleistung. Unter Gedächtnisleistung wird die Fähigkeit des Gehirns verstanden, Informationen aufzunehmen, abzuspeichern und zu einem späteren Zeitpunkt wieder abrufen zu können. Die zu speichernden Informationen können vielfältig sein. Dabei kann es sich etwa um Eindrücke, Erlebnisse und Erfahrungen handeln, aber auch um Lernstoff, Zahlen, Namen, Fakten und aktuelle Dinge.

Der Begriff des Lernens beschreibt den Vorgang der Aneignung bzw. Änderung von Verhaltensweisen oder kognitiven Strukturen. Lernen kann nicht direkt beobachtet werden, sondern nur die Lernleistung, die während oder nach einem Lernvorgang gezeigt wird. Man spricht von Lernen, wenn ein Subjekt infolge mehr oder weniger passiv gemachter Erfahrung und/oder eigener Aktivität (und ihrer Konsequenzen) sein Verhalten in einer bestimmten Situation (einem bestimmten Reiz gegenüber) mehr als nur vorübergehend ändert.

Des Weiteren wird im Sinne der vorliegenden Erfindung die mentale Leistungsfähigkeit auch mit Menschen und Tieren assoziiert, die eine Demenzerkrankung aufweisen. Demenz ist der Oberbegriff für Erkrankungsbilder, die mit einem Verlust der mentalen Leistungsfähigkeit wie Denken, Erinnern, Orientieren und Verknüpfen von Denkinhalten, also mit der Gedächtnis- und Lernleistung, einhergehen und die dazu führen, dass alltägliche Aktivitäten nicht mehr eigenständig durchgeführt werden können. Dazu zählen unter anderem die Alzheimer-Krankheit, die Vaskuläre Demenz, Morbus Pick und Frontotemporale Demenz.

Im Sinne der vorliegenden Erfindung wird als Steigerung der mentalen Leistungsfähigkeit auch eine Verbesserung oder auch die Wiederherstellung der mentalen Leistungsfähigkeit verstanden.

Im Sinne der vorliegenden Erfindung können die Nahrungsergänzungsmittel Aminosäuren sowie leistungssteigernde Nahrungsergänzungsmittel wie Koffein umfassen.

Synergistische oder additive Enhancer können im Sinne der vorliegenden Erfindung Koffein, Nikotin oder eines der oben genannten bekannten Produkte zur Gedächtnissteigerung sein.

Weiterhin können im Sinne der vorliegenden Erfindung die Verbindungen zur Verbesserung von Aufnahme und Transport ausgewählt sein aus der Gruppe bestehend aus Liposomen, Cyclodextrinen, Lysin, Polyethylenglykol, Cholsäure und Phytosterole.

Mittel zur Verminderung der Spaltung oder Metabolisierung können im Sinne der vorliegenden Erfindung Inhibitoren der Esterhydrolyse, Inhibitoren des Effluxes aus Zellen, Inhibitoren der Metabolisierung in der Leber, z.B. Inhibitoren oxidierender Enzyme, sein.

Die vorliegende Erfindung wird mit den beigefügten Figuren bzw. Zeichnungen näher erläutert. Es zeigt:
Fig. 1A eine Übersicht eines Lernexperiments nach Verfütterung von Ferulasäureeicosanylester an *Drosophila melanogaster* Larven;
Fig. 1B eine graphische Darstellung, welche die Ergebnisse des Lernexperiments gemäß Fig. 1A darstellt;
Fig. 2A eine Übersicht eines Lernexperiments nach Verfütterung von Ferulasäureeicosanylester oder dessen Derivate an *Drosophila melanogaster* Larven;
Fig. 2B eine graphische Darstellung, welche die Ergebnisse des Lernexperiments gemäß Fig. 2A darstellt;
Fig. 3A eine Übersicht eines Lernexperiments nach Verfütterung von Ferulasäureeicosanylester an älteren *Drosophila melanogaster* Fliegen;
Fig. 3B eine graphische Darstellung, welche die Ergebnisse des Lernexperiments gemäß Fig. 3A darstellt;
Fig. 4A eine Übersicht eines Lernexperiments bei Mäusen nach Injektion von Ferulasäureeicosanylester;
Fig. 4B eine graphische Darstellung, welche die Ergebnisse des Lernexperiments gemäß Fig. 4A darstellt;
Fig. 5A Darstellungen von *ex vivo* elektrophysiologischen Ableitungen an hippocampalen CA1 Mauszellen, nach deren Behandlung mit Ferulasäureeicosanylester;
Fig. 5B eine graphische Darstellung, welche die Anzahl der Aktionspotentiale der CA1 pyramidalen Zellen gemäß Fig. 5A in Bezug auf die injizierte Stromstärke darstellt.

Die Verbindungen gemäß der allgemeinen Formel I sind lipophile Ester der Zimtsäure und deren Derivate, insbesondere Ferulasäurederivate. Zimtsäureester werden im Folgenden mit "ZSE" und Ferulasäureester mit "FSE" bezeichnet.

Diese Verbindungen haben gegenüber den im Stand der Technik bekannten Substanzen den Vorteil, dass sie spezifisch und signifikant die mentale Leistungsfähigkeit bei Menschen und Tieren steigern können. Somit kann auch die Aufmerksamkeit und Konzentration gesteigert werden. Weiterhin vorteilhaft ist, dass eine genaue Dosierung möglich ist und somit eine Schwankung in der Effektivität ausgeschlossen werden kann.

Weiterhin vorteilhaft ist somit, dass diese Verbindungen auch für die Behandlung von Demenz, insbesondere der Alzheimer-Krankheit angewendet werden können. Demenz zeichnet sich insbesondere dadurch aus, dass ein Verlust der mentalen Leistungsfähigkeit einhergeht. Demnach würde die Steigerung der mentalen Leistungsfähigkeit durch Anwendung der Verbindungen gemäß der vorliegenden Erfindung auch vorteilhaft für die Behandlung von Demenz sein.

Die pharmazeutische Zusammensetzung der vorliegenden Erfindung wird mit den üblicherweise verwendeten pharmazeutisch-technischen Hilfsstoffen und Zusatzstoffen entsprechend der gewünschten Applikationsart in einer geeigneten Dosierung in bekannter Weise hergestellt.

Bevorzugt ist die pharmazeutische Zusammensetzung zur oralen, intranasalen oder subkutanen Verabreichung hergerichtet.

Darreichungsformen der pharmazeutischen Zusammensetzung sind beispielsweise Tabletten, Lutschtabletten, Filmtabletten, Dragees, Kapseln, Pillen, Pulver, Lösungen, Aerosole, Suspensionen, Depotformen oder parenterale Zubereitungen wie Injektionslösungen. Insbesondere sind für die orale Applikation solche Darreichungsformen bevorzugt, welche als Nahrungs- und/oder Futtermittel, Getränke oder Nahrungsergänzungsmittel verabreicht werden können.

Die Herstellung der erfindungsgemäßen pharmazeutischen Zubereitungen ist an sich bekannt und in den dem Fachmann bekannten Handbüchern beschrieben, beispielsweise Hager's Handbuch (5.) 2, 622-1045; List et al., Arzneiformenlehre, Stuttgart: Wiss. Verlagsges. 1985; Sucher et al. Pharmazeutische Technologie, Stuttgart: Thieme 1991; Ullmann's Technologie, Berlin: Ullstein Mosby 1995.

Die Erfinder haben überraschenderweise gefunden, dass die Anwendung einer Verbindung gemäß der Formel I bei *Drosophila melanogaster* sowie in Mäusen, zu einer signifikanten Steigerung der mentalen Lernleistungen führt.

Die Zugabe von Ferulasäureeicosanylester (FSE-20) in das Futter von Larven der Taufliege *Drosophila melanogaster* steigert deren Lern- und Gedächtnisleistung signifikant und dosisabhängig. Weiterhin wurde auch gefunden, dass auch die Lernleistung von älteren Fliegen verdoppelt wird. Andere synthetisierte FSEs führen ebenfalls zu Lernsteigerungen bei der Drosophila-Larve, jedoch ist die Verbesserung der Lernleistung bei signifikanter Verkürzung der Alkylkette der Alkoholkomponente der Ester (Effekt von FSE-20 > FSE-16 >> FSE-8), oder bei Hydrierung der Doppelbindung zu 2,3-Dihydroferulasäure (DH-FSE-20) weniger ausgeprägt. Langkettige Ester der para-Kumarsäure (4-Hydroxyzimtsäure (4OH-ZSE-20)), denen gegenüber den Ferulasäureestern die Methoxygruppe fehlt, zeichnen sich durch eine schwächere aber immer noch deutliche Aktivität aus. Weiterhin haben die Erfinder gefunden, dass die aktivsten Verbindungen schon im nanomolaren Bereich wirken. Zum Beispiel wirkt FSE-20 bei 700 nM im Futterbrei.

Des Weiteren haben die Erfinder gefunden, dass bei Mäusen die Injektion von FSE-20 vor dem Lernexperiment zu einem über die Zeit stabileren Gedächtnis führt. Außerdem zeigen *ex-vivo* Versuche, dass die Erregbarkeit von pyramidalen CA1 Hippocampus-Zellen, durch die Behandlung von FSE-20 in Mäusen, dosisabhängig zunimmt. Es ist bekannt, dass die pyramidalen CA1 Hippocampus-Zellen, sowohl bei Nagern als auch dem Menschen, an Lern- und Gedächtnisleistungen beteiligt sind.

Da die überraschenderweise gefundenen Wirkungen auf der Verabreichung einer Einzelsubstanz beruhen, wird im Gegensatz zur Applikation eines Pflanzenextraktes eine exakte Dosierung und Vermeidung von Überdosierungen der bioaktiven Substanz gewährleistet.

Eine Übertragbarkeit auf den Menschen ist gegeben, da die neuromolekularen Mechanismen des Lernens und des Gedächtnisses bei Mensch und Tier, einschließlich der hier verwendeten Taufliege, in sehr hohem Maß ähnlich sind. Zudem besitzen FSE-20 injizierte Mäuse ein stabileres Gedächtnis und *ex vivo* Versuche an hippocampalen CA1-Zellen zeigen eine Erhöhung der Erregbarkeit dieser Zellen, wenn sie mit FSE-20 Lösung behandelt wurden. Da eine Abnahme an zellulärer Erregbarkeit mit dem Abbau kognitiver Fähigkeiten im Alter und bei Demenz assoziiert ist, weist auch dies darauf hin, dass FSE-20 einige Gedächtnisformen fördert, indem es die Zellerregbarkeit erhöht.

Die folgenden Verbindungen sind daher, gemäß der vorliegenden Erfindung, besonders bevorzugte Ausführungsformen:

| | |
|---|---|
| Icosyl-(2E)-3-(4-hydroxy-3-methoxyphenyl)-prop-2-enoat (FSE-20) | 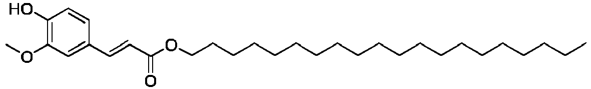 |
| Octyl-(2E)-3-(4-hydroxy-3-methoxyphenyl)-prop-2-enoat (FSE-8) | 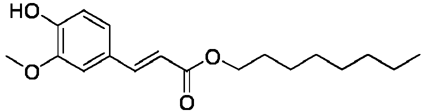 |
| Hexadecyl-(2*E*)-3-(4-hydroxy-3-methoxyphenyl)-prop-2-enoat (FSE-16) | 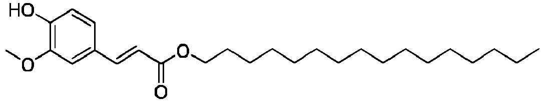 |
| Icosyl-(2*E*)-3-(4-hydroxyphenyl)-prop-2-enoat (4OH-ZSE-20) | 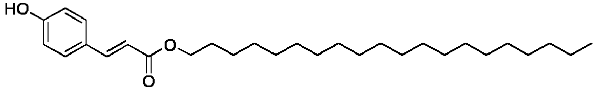 |
| Icosyl-3-(4-hydroxy-3-methoxyphenyl)-propanoat (DH-FSE-20) | 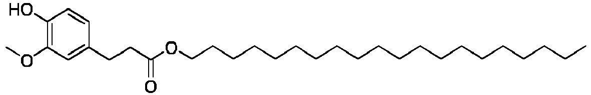 |
| Dodecyl-(2E)-3-(4-hydroxy-3-methoxyphenyl)-prop-2-enoat (FSE-12) | 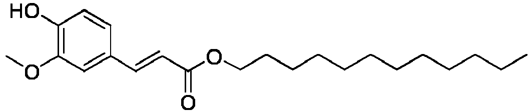 |

Nachfolgend wird die vorliegende Erfindung noch einmal mit anderen Worten wiedergegeben:
Der Gegenstand der vorliegenden Erfindung betrifft die Anwendung von Estern der allgemeinen Formel IA 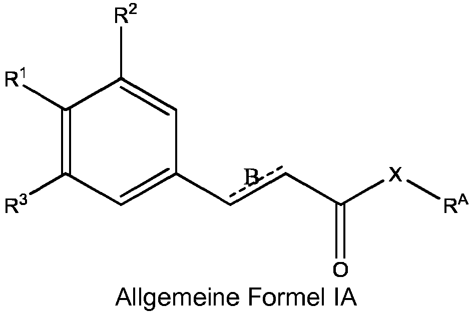 mit
B = Einfach-, Doppel- (E oder Z), oder Dreifach-Bindung, wobei eine E-Doppelbindung bevorzugt ist;
X = O, S, NH, N-Me, N-Et, N-Pr, N-R^{A}; wobei O bevorzugt ist;
R^{A} = Lipophile Kette, bevorzugt C₆ - C₆₀-Alkyl, C₆ - C₆₀-Alkenyl, C₆ - C₆₀-Polyalkenyl, oder C₆ - C₆₀-Isoalkyl, C₆ - C₆₀-PEG-OH oder C₆ - C₆₀-PEG-OMe, Oligoprenyl (C₅-C₁₂₀) und besonders bevorzugt C₁₂ - C₂₈-Alkyl,
R1 = OH; R²; R³ = H, OMe, OEt, OPr in jeglicher Kombination;
wobei Ferulasäurederivate (R¹ = OH, R² = OCH₃, R³ = H) ganz besonders bevorzugt sind und / oder Kombinationen (Mischungen) der obigen Verbindungen zur Steigerung der generellen mentalen Leistungsfähigkeit bei Mensch und Tier, bevorzugt zur Steigerung der Lern- und/oder Gedächtnisleistung bei Mensch und Tier. Weiterhin bevorzugt ist die Anwendung von Estern der allgemeinen Formel IA zur Behandlung bei Demenzerkrankungen, im Speziellen die Alzheimer-Krankheit.

Weiterhin wird die Anwendung von Ferulassäureestern langkettiger unverzweigter und / oder einfach methylverzweigter Alkohole (R^{A}), auch in jeglichen Mischungen, bevorzugt. Insbesondere werden dabei die natürlich vorkommenden langkettigen Zimtsäurealkylester, hier insbesondere die Ferulasäureester des Tetracosanols, Docosanols, Heneicosanol, Eicosanols, Nonadecanols, Octadecanols, Hexadecanols, Dodecanols bevorzugt. Die Anwendung der Ferulasäureester mit R^{A} = C₁₆-C₂₄ ist bevorzugt, ganz besonders des Ferulasäure-eicosanylesters (C₂₀-Ester).

Weiterhin ist die Verwendung der zuvor genannten Verbindungen in Nahrungs- und/oder Futtermitteln inkl. Getränken und/oder Nahrungsergänzungsmitteln und/oder pharmazeutischen Präparationen jeglicher Art bevorzugt.

Die Verwendung der zuvor genannten Substanzen und Präparationen in der Form von Prodrugs, insbesondere solche in denen eine phenolische Gruppe durch eine zeitnah oder biotisch spaltbare Gruppe geschützt ist, z. B. als phenolischer Ester, besonders das Acetat oder Succinat der Ferulasäureester, ist außerdem von Vorteil (nicht Gegenstand der Erfindung).

Weiterhin ist die Verwendung der zuvor genannten Verbindungen auch in Kombination mit anderen Wirkstoffen, insbesondere leistungssteigernden Stoffen (z.B. Koffein) und/oder Nahrungsergänzungsmitteln inkl. Aminosäuren und/oder Matrizes und/oder synergistischen Enhancern und/oder Präparationen zur Verbesserung von Aufnahme oder Transport (z.B. Liposomen, Cyclodextrine, Lysin, PEGs, Cholsäuren und Phytosterole oder andere lipophile Träger) und/oder Mitteln oder Verkapselungen zur Verminderung der Spaltung oder Metabolisierung, bevorzugt. Diese Auflistung stellt jedoch lediglich bevorzugte Ausführungen dar und soll keine Beschränkung auf die genannten Wirkstoffe, Mittel und Präparationen sein.

Die Verwendung der zuvor genannten Substanzen und Präparationen ist besonders für die nichtorale Anwendung, besonders die Verwendung in Nasalsprays oder in Produkten zur transdermalen Applikation, bevorzugt.

Die nachfolgenden Beispiele erläutern die Erfindung näher, ohne den Umfang der Erfindung zu beschränken.

### Beispiel 1:

Steigerung der Gedächtnisleistung von *Drosophila melanogaster* Larven durch Verfütterung von synthetisiertem FSE-20.

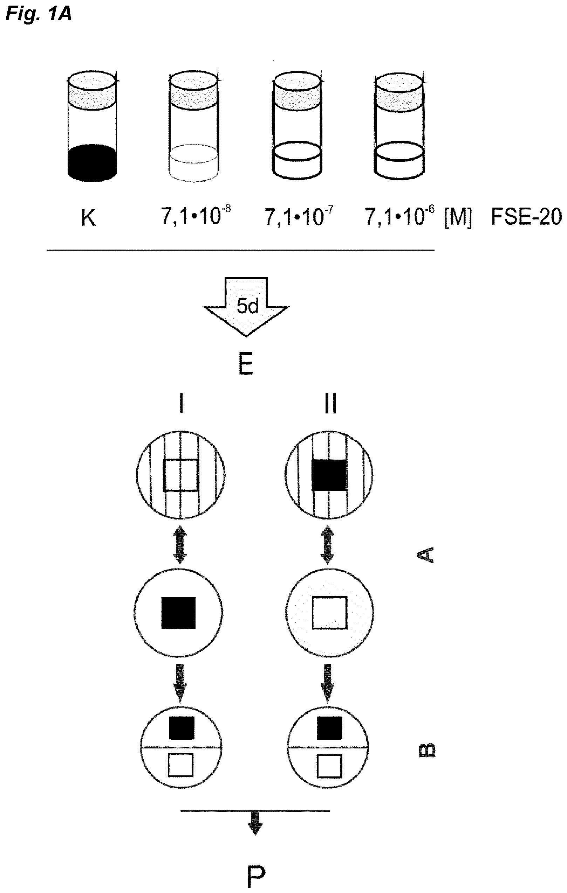

In Figur 1A wird eine Übersicht eines Lernexperiments E mit *Drosophila melanogaster* Larven dargestellt. Die Übersicht zeigt, dass die Larven in vier Gruppen aufgeteilt wurden. Eine Gruppe stellt die Kontrollgruppe K dar, welche mit Standard-Futterbrei aufgezogen wurde und die drei anderen Gruppen wurden jeweils mit verschiedenen Konzentrationen von FSE-20 (7,1 × 10⁻⁸, 7,1 × 10⁻⁷ und 7,1 × 10⁻⁶ [M] FSE-20) im Futterbrei aufgezogen. Nach 5 Tagen (Pfeil mit "5d") erfolgte das Lernexperiment E.

In dem Lernexperiment E wurden die Tiere, je einem von zwei reziproken Trainingsregimen A unterzogen und dann einem Test B unterzogen. Der ersten Gruppe I wurde im Training A ein erster Duft mit der Futterbelohnung (Fruktose) präsentiert, während ein zweiter Duft alleine präsentiert wurde (erster Duft+/zweiter Duft). In der Figur 1A wird der erste Duft als weißes Quadrat und der zweite Duft als schwarzes Quadrat dargestellt. Des Weiteren wird durch einen weißen Kreis mit Längsstreifen eine Petrischale mit Fruktose und durch einen weißen Kreis ohne die Längsstreifen eine Petrischale ohne Fruktose dargestellt. Die zweite Gruppe II wurde reziprok trainiert (erster Duft /zweiter Duft+). Im Test B des Lernexperiments E wurde anschließend untersucht, ob die Larven beider Gruppen I, II in einer Wahlsituation den ersten Duft oder den zweiten Duft bevorzugen und somit ihre Lern- und Gedächtnisleistung (Performance Index) P bestimmt.

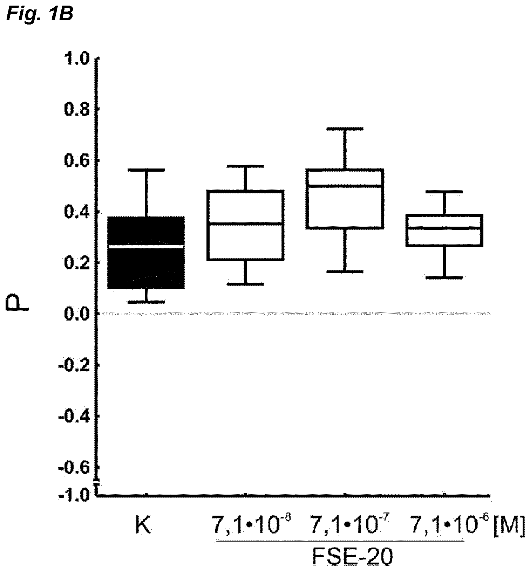

In der Figur 1B wird eine graphische Darstellung gezeigt, welche die Ergebnisse der Lern- und Gedächtnisleistung P der in dem Lernexperiment E getesteten Larven darstellt. Verglichen wird somit die Lern- und Gedächtnisleistung P von Larven, die entweder mit Standard-Futterbrei (Kontrolle K, schwarze Box) oder mit 7,1 × 10⁻⁸ 7,1 × 10⁻⁷, oder 7,1 × 10⁻⁶ M FSE-20 Lösung (weiße Boxen) im Futterbrei aufgewachsen sind. Dieser graphischen Darstellung ist zu entnehmen, dass die Lern- und Gedächtnisleistung P aller Larvengruppen, die mit FSE-20 Lösungen im Futterbrei aufgewachsen sind, im Vergleich zu der Kontrollgruppe K gesteigert ist. Weiterhin ist der graphischen Darstellung zu entnehmen, dass sich bei sehr niedrigen Konzentrationen (70 nM) eine lernsteigernde Tendenz zeigt. Bei 700 nM hat sich die Lern- und Gedächtnisleistung P sogar erheblich, um ca. 50 %, verbessert, welche jedoch bei sehr hohen Konzentrationen ab 7 µM wieder abnimmt.

### Beispiel 2:

Vergleich der Lern- und Gedächtnisleistung P von *Drosophila melanogaster* Larven nach Verfütterung von synthetisiertem FSE-20 oder dessen Derivate.

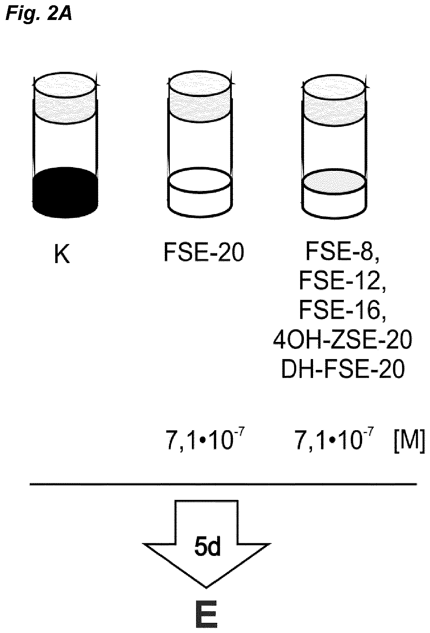

In der Figur 2A wird eine Übersicht mit den in diesem Beispiel als Zugabe in den Futterbrei verwendeten Verbindungen gezeigt. Eine Gruppe der *Drosophila melanogaster* Larven war die Kontrollgruppe K, welche mit Standard-Futterbrei aufgezogen wurde. Des Weiteren wurde eine Gruppe mit FSE-20 in einer Konzentration von 7,1 × 10⁻⁷ M im Futterbrei aufgezogen. Die anderen Gruppen wurden mit einer Konzentration von 7,1 × 10⁻7 M der unterschiedlichen FSE-20 Derivate im Futterbrei aufgezogen. Diese FSE-20 Derivate umfassen FSE-8, FSE-12, FSE-16, 4OH-ZSE-20 und DH-FSE-20. Nach fünf Tagen (Pfeil mit "5d") werden die *Drosophila melanogaster* Larven in dem Lernexperiment E, wie in Beispiel 1 beschrieben, getestet.

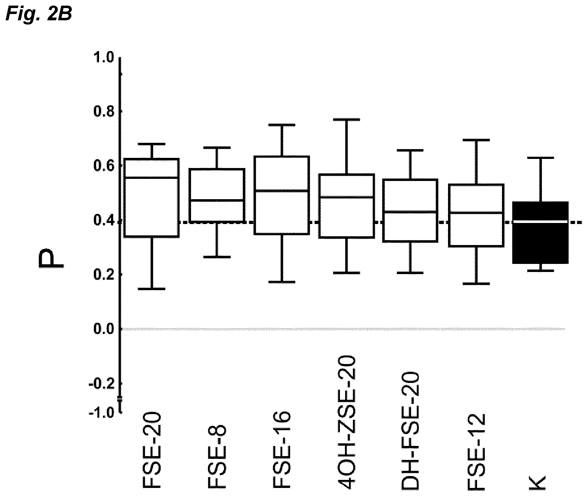

In Figur 2B wird eine graphische Darstellung gezeigt, welche die folgenden Ergebnisse des Lernexperiments E zusammenfasst:
Die Kontrollgruppe K zeigt keine Steigerung in der Lern- und Gedächtnisleistung P. Wohingegen die Zugabe von FSE-20 im Futterbrei eine deutliche Steigerung der Lern- und Gedächtnisleistung P in den Larven hervorruft.

Auch die Derivate von FSE-20 steigern, wie nachfolgend zusammengefasst, die Lern- und Gedächtnisleistung P der Larven.

Es wurde gezeigt, dass FSE-8 die Lern- und Gedächtnisleistung P von *Drosophila* Larven steigert. Hierzu wurde die Substanz mit einer finalen Konzentration von 700 nM in den Futterbrei gemischt. Weiterhin wurde gezeigt, dass sich FSE-16, bei einer finalen Konzentration von 700 nM im Futterbrei, auch lernsteigernd auf die Tiere auswirkt. Auch die Zugabe von DH-FSE-20 in einer finalen Konzentration von 700 nM im Futterbrei hat einen lern- und gedächtnissteigernden Effekt auf die Larven.

Die Zugabe von 4OH-ZSE-20 in den Futterbrei der *Drosophila melanogaster* Larven, bei einer Konzentration von 700 nM, zeigt eine geringfügige Steigerung der Lern-und Gedächtnisleistung P der Tiere. Auch die Zugabe von FSE-12 in einer finalen Konzentration von 700 nM im Futterbrei ruft nur eine schwache Steigerung der Lern- und Gedächtnisleistung P in den Tieren hervor.

### Beispiel 3

Steigerung der Lern- und Gedächtnisleistung P von erwachsenen, älteren *Drosophila melanogaster* Fliegen mittels synthetisiertem FSE-20.

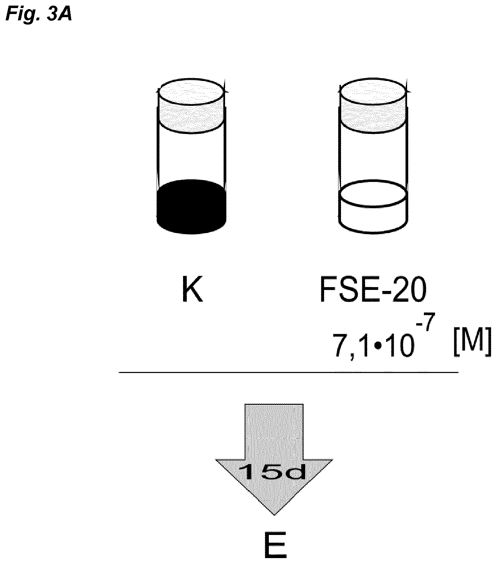

Figur 3A zeigt eine Übersicht der Durchführung des Lernexperiments E mit älteren Fliegen. In diesem Experiment wurden die Fliegen in zwei Gruppen unterteilt, eine Kontrollgruppe K, welche mit Standard-Futter gefüttert wurde und eine Testgruppe, bei der in das Futter FSE-20 zugegeben wurde. Die Konzentration des FSE-20 im Futter betrug 7,1 × 10⁻⁷ M. Die Fliegen wurden 15 Tage (Pfeil mit "15d") nach dem Schlüpfen der adulten Tiere in einem Lernexperiment E, welches dem vom Prinzip in Beispiel 1 entspricht, getestet.

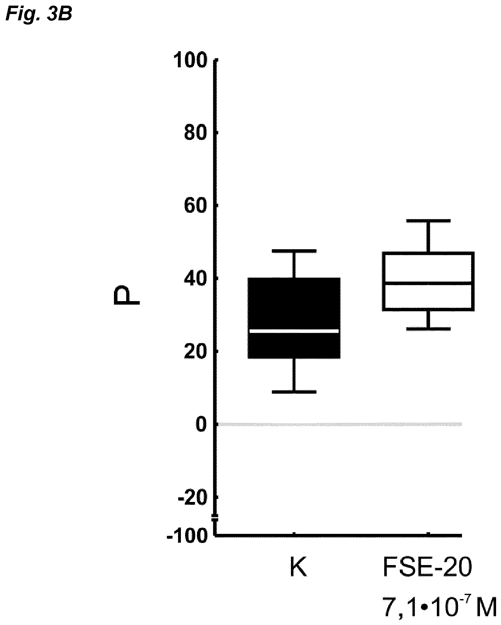

Die Figur 3B zeigt eine graphische Darstellung, welche die Ergebnisse des Lernexperiments E darstellt. Es zeigt sich, dass sich die Lern- und Gedächtnisleistung P bei älteren Tieren, die FSE-20 im Fliegenfutter hatten, erheblich um bis zu ca. 50 % verbessert.

### Beispiel 4

Lernexperiment E mit Mäusen nach der Injektion von FSE-20.

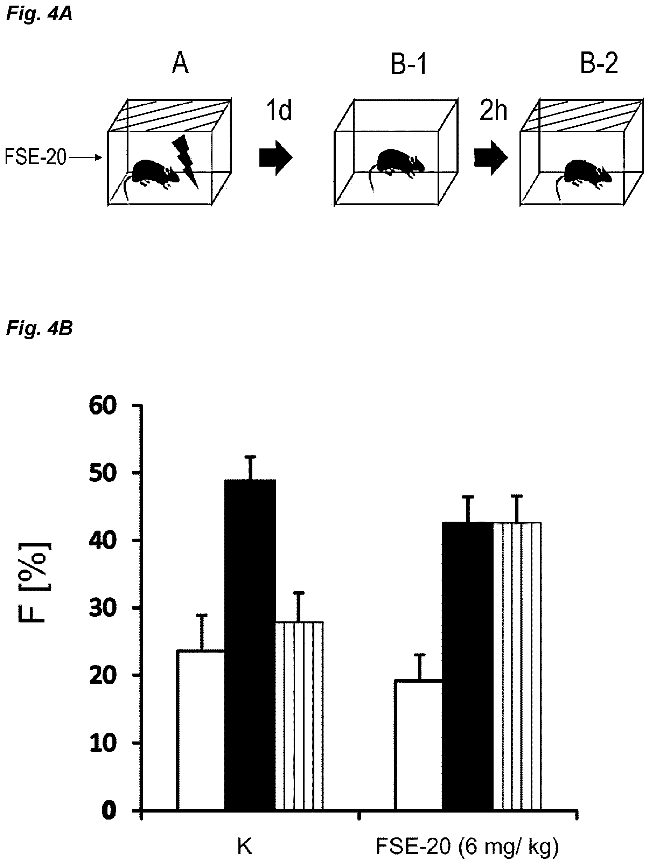

Figur 4A zeigt eine Übersicht des Lernexperiments E bei Mäusen. Um den Effekt von FSE-20 auf die Lern- und Gedächtnisleistung P bei 3 bis 4 Monate alten Mäusen zu bestimmen, wurde den Tieren eine FSE-20 Lösung (6 mg/kg Körpermasse) intraperitoneal vor dem Training A injiziert. Im Lernexperiment E am nächsten Tag wurden die Tiere beim Training A in eine bestimmte Umgebung gesetzt, während den Mäusen gleichzeitig ein aversiver elektrischer Reiz (Blitzsymbol) präsentiert wurde. Im ersten Test B-1 wurde anschließend ein Tag später (1d) die unspezifische Furchtreaktion (Freezing) F in einer neutralen, das heißt in einer für die Tiere neuen Umgebung gemessen. Im zweiten Test B-2 wurde zwei Stunden (2h) nach dem ersten Test B-1, die Furchtreaktion in der Umgebung gemessen, in der die Tiere den elektrischen Reiz während des Trainings A präsentiert bekamen (konditionierte Umgebung).

In der Figur 4B wird eine graphische Darstellung gezeigt, welche die Ergebnisse des Lernexperiments E an den Mäusen zusammenfasst. Dargestellt ist der Anteil der Zeit in der die zwei Mäusegruppen (Kontrolle K und FSE-20 behandelte Gruppe) eine Furchtreaktion (Freezing) F zeigten, die während des ersten Tests B-1 in der neutralen, das heißt für die Tiere neuen Umgebung (weißer Balken) und dann in dem zweiten Test B-2 in zwei aufeinanderfolgenden Zeitspannen innerhalb der konditionierten Umgebung gemessen wurde. Die erste Zeitspanne des zweiten Tests umfasst die 1. - 5. Minute und die zweite Zeitspanne umfasst die 6. - 10. Minute. Das Ergebnis der ersten Zeitspanne ist als schwarzer Balken und das Ergebnis der zweiten Zeitspanne als weißer Balken mit schwarzen Längsstreifen dargestellt. Bei FSE-20 behandelten Mäusen zeigt sich in der zweiten Hälfte des Lernexperiments E (6. - 10. Min) ein genauso stark ausgeprägtes Furchtveralten F wie in der ersten Hälfte (1. - 5. Min), während die Kontrolle K in der zweiten Hälfte ein stark verringertes Furchtverhalten F zeigt. Demzufolge hat zu diesem Zeitpunkt bei der Kontrollgruppe K bereits Gedächtnisverlust bzw. Extinktion eingesetzt, wogegen die FSE-20 behandelten Tiere ein über die Zeit stabileres Gedächtnis haben.

### Beispiel 5

*Ex vivo* elektrophysiologische Ableitungen an hippocampalen CA1 Mauszellen mit FSE-20.

An ganzen CA1 Pyramidenzellen aus "akuten" (frisch präparierten) Hippocampusschnitten, von vier bis sechs Wochen alten männlichen Mäusen, wurde die Patch-Clamp-Technik durchgeführt, um die Effekte von FSE-20 auf die neuronale Erregbarkeit zu messen.

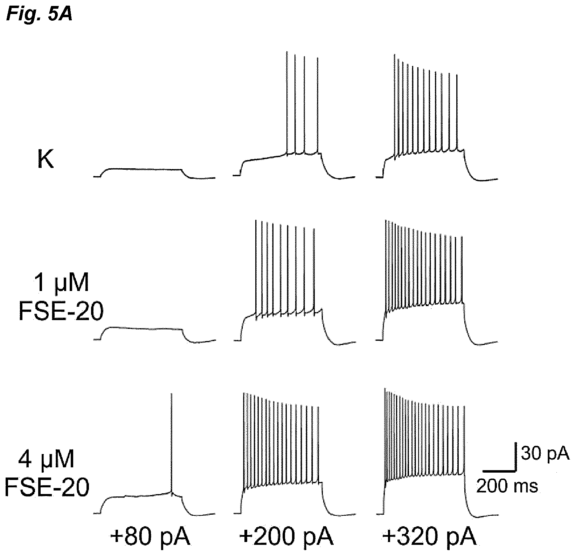

Figur 5A zeigt Darstellungen der repräsentativen Reaktion der CA1-Zellen von Aktionspotentialen auf drei aufeinanderfolgende elektrische Stimulationen von + 80, + 200 und + 320 pA. Es werden die Aktionspotentiale der Kontrolle K sowie die von den mit FSE-20 behandelten Zellen gezeigt. Die mittlere Darstellung zeigt die Aktionspotentiale der mit 1 µM FSE-20 behandelten Zellen und die unterste Darstellung zeigt die Aktionspotentiale der mit 4 µM FSE-20 behandelten Zellen.

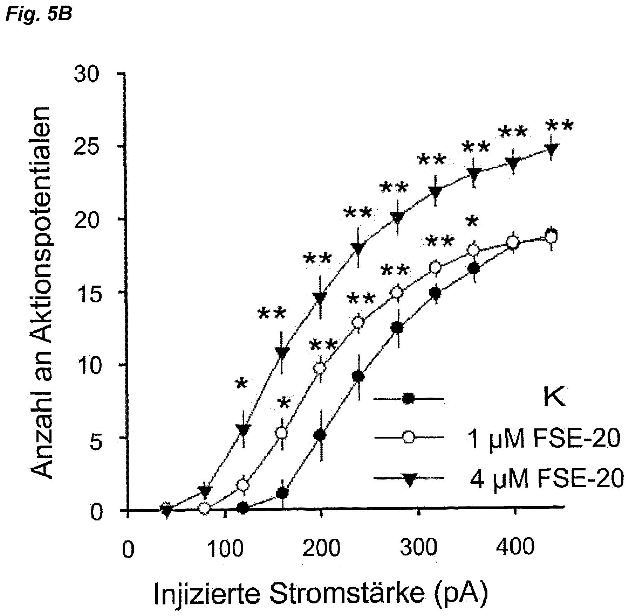

In Figur 5B wird eine graphische Darstellung, in der die Anzahl der Aktionspotentiale in Bezug auf die injizierte Stromstärke (pA) der unterschiedlich behandelten Zellen und der Kontrollgruppe K dargestellt werden.

Die Anzahl an Aktionspotentialen, die als Antwort auf Stromstärken von 40 bis 440 pA generiert wurden, sind in beiden FSE-20 behandelten Gruppen (1 µM und 4 µM) im Vergleich zur Kontrolle K signifikant erhöht. Somit weist die höhere Zellerregbarkeit in Anwesenheit von FSE-20 darauf hin, dass FSE-20 einige Gedächtnisformen fördert, da eine Abnahme an zellulärer Erregbarkeit hingegen mit dem Abbau kognitiver Fähigkeiten, im Alter und bei Demenz, assoziiert wird.

### Bezugszeichenliste

- A: Training
- B: Test
- E: Lernexperiment
- F: Furchtreaktion
- K: Kontrolle oder Kontrollgruppe
- P: Lern- und Gedächtnisleistung (Performance Index)

- I: erste Gruppe
- II: zweite Gruppe

## Patentansprüche

1. Verbindung gemäß der allgemeinen Formel I 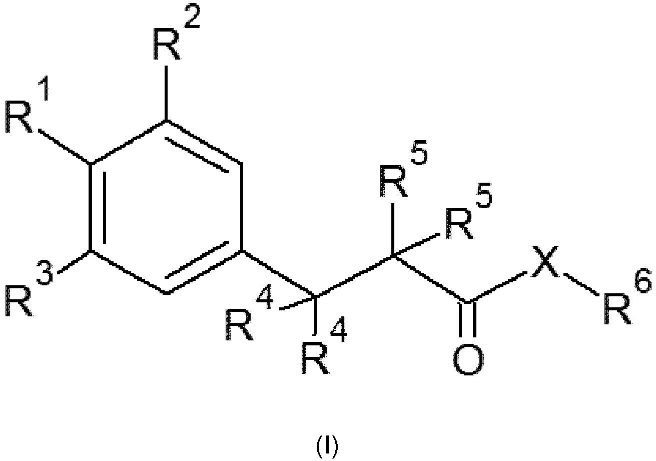 wobei
R¹ Hydroxy ist,
R² ausgewählt ist aus der Gruppe bestehend aus H, Methoxy, Ethoxy, n-Propoxy und i-Propoxy;
und
R³ ausgewählt ist aus der Gruppe bestehend aus H, Methoxy, Ethoxy, n-Propoxy und i-Propoxy;
R⁴ und R⁵ jeweils unabhängig voneinander H sind oder jeweils ein R⁴ und ein R⁵ zusammen eine C-C-Bindung bilden, derart, dass zwischen den einander benachbarten C-Atomen, an welchen sich R⁴ und R⁵ befinden, eine Einzel-, Doppel- oder Dreifachbindung vorliegt;
R⁶ ausgewählt ist aus der Gruppe bestehend aus unverzweigten oder verzweigten C₆ bis C₆₀-Alkyl, unverzweigten oder verzweigten C₆ bis C₆₀-Alkenyl, C₆ bis C₆₀-Hydroxypolyethylenglykol, C₆ bis C₆₀-Methoxypolyethylenglykol und C₅ bis C₁₂₀-Prenyl; und
X ausgewählt ist aus der Gruppe bestehend aus O, S, N-H, N-Methyl, N-Ethyl, N-n-Propyl, N-i-Propyl und N-R⁶; deren Stereoisomere, Enantiomere oder Diastereomere, deren physiologisch annehmbaren Salze sowie Mischungen der genannten Verbindungen zur therapeutischen Anwendung bei der Steigerung von mentaler Leistungsfähigkeit.

2. Verbindung zur therapeutischen Anwendung bei der Steigerung von mentaler Leistungsfähigkeit, gemäß Anspruch 1, **dadurch gekennzeichnet, dass** R² Methoxy ist.

3. Verbindung zur therapeutischen Anwendung bei der Steigerung von mentaler Leistungsfähigkeit, gemäß einem der Ansprüche 1 oder 2, **dadurch gekennzeichnet, dass** R³ H ist.

4. Verbindung zur therapeutischen Anwendung bei der Steigerung von mentaler Leistungsfähigkeit, gemäß einem der Ansprüche 1 bis 3, **dadurch gekennzeichnet, dass** die durch R⁴ und R⁵ gebildete C-C-Bindung eine Doppelbindung ist.

5. Verbindung zur therapeutischen Anwendung bei der Steigerung von mentaler Leistungsfähigkeit, gemäß Anspruch 4, **dadurch gekennzeichnet, dass** die Doppelbindung eine E-Doppelbindung ist.

6. Verbindung zur therapeutischen Anwendung bei der Steigerung von mentaler Leistungsfähigkeit, gemäß einem der Ansprüche 1 bis 5, **dadurch gekennzeichnet, dass** X O ist.

7. Verbindung zur therapeutischen Anwendung bei der Steigerung von mentaler Leistungsfähigkeit, gemäß einem der Ansprüche 1 bis 6, **dadurch gekennzeichnet, dass** R⁶ ein C₈ bis C₂₈-Alkyl, bevorzugt ein C₁₂ bis C₂₄-Alkyl und besonders bevorzugt ein C₈-Alkyl, ein C₁₂-Alkyl, ein C₁₆-Alkyl oder ein C₂₀-Alkyl ist.

8. Verbindung zur therapeutischen Anwendung bei der Steigerung von mentaler Leistungsfähigkeit, gemäß Anspruch 1, **dadurch gekennzeichnet, dass** die Verbindung ausgewählt ist aus der Gruppe bestehend aus Icosyl-(2*E*)-3-(4-hydroxy-3-methoxyphenyl)-prop-2-enoat, Octyl-(2*E*)-3-(4-hydroxy-3-methoxyphenyl)-prop-2-enoat, Hexadecyl-(2*E*)-3-(4-hydroxy-3-methoxyphenyl)-prop-2-enoat, Icosyl-3-(4-hydroxy-3-methoxyphenyl)-propanoat, Dodecyl-(2*E*)-3-(4-hydroxy-3-methoxyphenyl)-prop-2-enoat und Icosyl-(2*E*)-3-(4-hydroxyphenyl)-prop-2-enoat.

9. Verbindung zur therapeutischen Anwendung bei der Steigerung von mentaler Leistungsfähigkeit, gemäß einem der Ansprüche 1 bis 8, wobei die mentale Leistungsfähigkeit die Gedächtnisleistung und die Lernleistung umfasst und mit einer Demenzerkrankung assoziiert ist.

10. Pharmazeutische Zusammensetzung zur therapeutischen Anwendung bei der Steigerung von mentaler Leistungsfähigkeit, umfassend mindestens eine Verbindung gemäß einem der Ansprüche 1 bis 9 sowie die physiologisch annehmbaren Salze und Stereoisomere, Enantiomere, Diastereomere oder Mischungen daraus und pharmazeutisch annehmbare Hilfsstoffe und Zusatzstoffe.

11. Pharmazeutische Zusammensetzung zur therapeutischen Anwendung bei der Steigerung von mentaler Leistungsfähigkeit, gemäß Anspruch 10, in Kombination mit weiteren Wirkstoffen, die ausgewählt sind aus der Gruppe bestehend aus Nahrungsergänzungsmitteln, synergistischen Enhancern, additiven Enhancern, Verbindungen zur Verbesserung von Aufnahme und Transport und Mitteln zur Verminderung der Spaltung oder Metabolisierung sowie deren Mischungen.

12. Pharmazeutische Zusammensetzung zur therapeutischen Anwendung bei der Steigerung von mentaler Leistungsfähigkeit, gemäß Anspruch 10 oder 11, **dadurch gekennzeichnet, dass** die pharmazeutische Zusammensetzung zur oralen, intranasalen oder subkutanen Verabreichung hergerichtet ist.

13. Nicht-therapeutische Anwendung einer Verbindung der Formel I wie in Anspruch 1 dargestellt,
wobei
R¹ Hydroxy ist,
R² ausgewählt ist aus der Gruppe bestehend aus H, Methoxy, Ethoxy, n-Propoxy und i-Propoxy; und
R³ ausgewählt ist aus der Gruppe bestehend aus H, Methoxy, Ethoxy, n-Propoxy und i-Propoxy;
R⁴ und R⁵ jeweils unabhängig voneinander H sind oder jeweils ein R⁴ und ein R⁵ zusammen eine C-C-Bindung bilden, derart, dass zwischen den einander benachbarten C-Atomen, an welchen sich R⁴ und R⁵ befinden, eine Einzel-, Doppel- oder Dreifachbindung vorliegt;
R⁶ ausgewählt ist aus der Gruppe bestehend aus unverzweigten oder verzweigten C₆ bis C₆₀-Alkyl, unverzweigten oder verzweigten C₆ bis C₆₀-Alkenyl, C₆ bis C₆₀-Hydroxypolyethylenglykol, C₆ bis C₆₀-Methoxypolyethylenglykol und C₅ bis C₁₂₀-Prenyl; und
X ausgewählt ist aus der Gruppe bestehend aus O, S, N-H, N-Methyl, N-Ethyl, N-n-Propyl, N-i-Propyl und N-R⁶;
deren Stereoisomere, Enantiomere oder Diastereomere, deren physiologisch annehmbaren Salze sowie Mischungen der genannten Verbindungen,
bei der Steigerung von mentaler Leistungsfähigkeit.

## Claims

1. A compound according to the general formula I 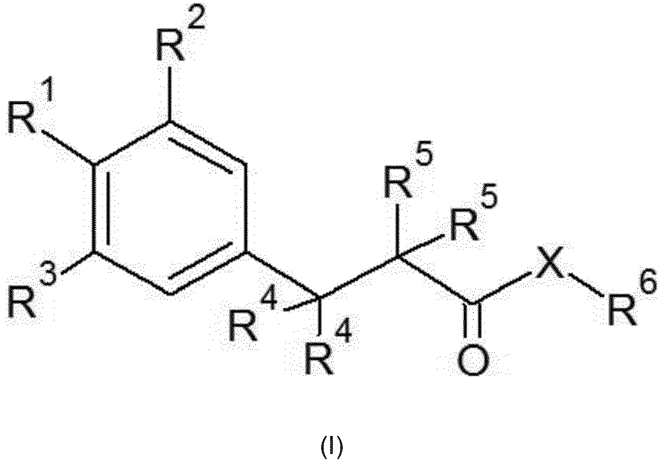 wherein
R¹ is hydroxy,
R² is selected from the group consisting of H, methoxy, ethoxy, n-propoxy and i-propoxy;
and
R³ is selected from the group consisting of H, methoxy, ethoxy, n-propoxy and i-propoxy;
R⁴ and R⁵, each independently of the other, are H or, in each case, an R⁴ and an R⁵ together form a C-C-bond in such a way that, between the mutually neighboring C atoms at which R⁴ and R⁵ are located, there is a single, double, or triple bond;
R⁶ is selected from the group consisting of unbranched or branched C₆ bis C₆₀ alkyl, unbranched or branched C₆ to C₆₀ alkenyl, C₆ to C₆₀ hydroxypolyethylene glycol, C₆ to C₆₀ methoxypolyethylene glycol, and C₅ to C₁₂₀ prenyl; and
X is selected from the group consisting of O, S, N-H, N-methyl, N-ethyl, N-n-propyl, N-i-propyl and N-R⁶;
the stereoisomers, enantiomers or diastereomers thereof, the physiologically acceptable salts thereof, as well as mixtures of the cited compounds for therapeutical use in the increasing of mental capability.

2. The compound for therapeutical use in the increasing of mental capability, according to claim 1, **characterized in that** R² is methoxy.

3. The compound for therapeutical use in the increasing of mental capability, according to one of claims 1 or 2, **characterized in that** R³ is H.

4. The compound for therapeutical use in the increasing of mental capability, according to one of claims 1 to 3, **characterized in that** the C-C-bond formed by R⁴ and R⁵ is a double bond.

5. The compound for therapeutical use in the increasing of mental capability, according to claim 4, charcterized in that the double bond is an E double bond.

6. The compound for therapeutical use in the increasing of mental capability, according to one of claims 1 to 5, **characterized in that** X is O.

7. The compound for therapeutical use in the increasing of mental capability, according to one of claims 1 to 6, **characterized in that** R⁶ is a C₈ to C₂₈ alkyl, preferably a C₁₂ to C₂₄ alkyl, and, especially preferred a C₈ alkyl, a C₁₂ alkyl, a C₁₆ alkyl or a C₂₀ alkyl.

8. The compound for therapeutical use in the increasing of mental capability, according to claim 1, **characterized in that** the compound is selected from the group consisting of icosyl-(2*E*)-3-(4-hydroxy-3-methoxyphenyl)-prop-2-enoate, octyl-(2*E*)-3-(4-hydroxy-3-methoxyphenyl)-prop-2-enoate, hexadecyl-(2*E*)-3-(4-hydroxy-3-methoxyphenyl)-prop-2-enoate, icosyl-3-(4-hydroxy-3-methoxyphenyl)-propanoate, dodecyl-(2*E*)-3-(4-hydroxy-3-methoxyphenyl)-prop-2-enoate, and icosyl-(2*E*)-3-(4-hydroxyphenyl)-prop-2-enoate.

9. The compound for therapeutical use in the increasing of mental capability, according to one of claims 1 to 8, wherein the mental capability comprises memory capability and learning capability and is associated with a dementia disease.

10. A pharmaceutical composition for therapeutical use in the increasing of mental capability, comprising at least one compound according to one of claims 1 bis 9 as well as the physiologically acceptable salts and stereosimers, enantioners, diastereomers, or mixtures thereof, and pharmaceutically acceptable excipients and additives.

11. The pharmaceutical composition for therapeutical use in the increasing of mental capability, according to claim 10, in combination with additional active substances, which are selected from the group consisting of nutritional supplements, synergistic enhancers, additive enhancers, compounds for improving absorption and transport, and agents for the reduction of cleavage or metabolization, as well as the mixtures thereof.

12. The pharmaceutical composition for therapeutical use in the increasing of mental capability, according to claims 10 or 11, **characterized in that** the pharmaceutical composition is prepared for oral, intranasal, or subcutaneous administration.

13. Non-therapeutical use of a compound according to formula (I) as indicated in claim 1,
wherein,
R¹ is hydroxy,
R² is selected from the group consisting of H, methoxy, ethoxy, n-propoxy and i-propoxy; and
R³ is selected from the group consisting of H, methoxy, ethoxy, n-propoxy and i-propoxy;
R⁴ and R⁵, each independently of the other, are H or, in each case, an R⁴ and an R⁵ together form a C-C-bond in such a way that, between the mutually neighboring C atoms at which R⁴ and R⁵ are located, there is a single, double, or triple bond;
R⁶ is selected from the group consisting of unbranched or branched C₆ bis C₆₀ alkyl, unbranched or branched C₆ to C₆₀ alkenyl, C₆ to C₆₀ hydroxypolyethylene glycol, C₆ to C₆₀ methoxypolyethylene glycol, and C₅ to C₁₂₀ prenyl; and
X is selected from the group consisting of O, S, N-H, N-methyl, N-ethyl, N-n-propyl, N-i-propyl and N-R⁶;
the stereoisomers, enantiomers or diastereomers thereof, the physiologically acceptable salts thereof, as well as mixtures of the cited compounds in the increasing of mental capability.

## Revendications

1. Composé selon la formule générale I 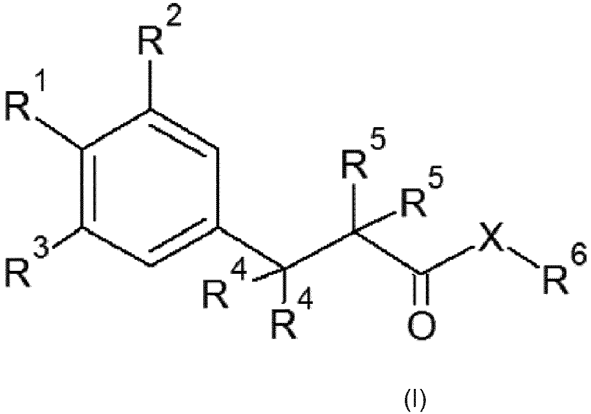 dans laquelle
R¹ est un hydroxy,
R² est choisi dans le groupe constitué de H, méthoxy, éthoxy, n-propoxy et i-propoxy;
et
R³ est choisi dans le groupe constitué par H, méthoxy, éthoxy, n-propoxy et i-propoxy;
R⁴ et R⁵ sont chacun indépendamment H ou un R⁴ et un R⁵ forment ensemble une liaison C-C, de telle sorte qu'il existe une liaison simple, double ou triple entre les atomes de C adjacents sur lesquels se trouvent R⁴ et R⁵;
R⁶ est choisi dans le groupe constitué par les groupes alkyle en C₆ à C₆₀ non ramifiés ou ramifiés, alcényle en C₆ à C₆₀ non ramifiés ou ramifiés, hydroxypolyéthylèneglycol en C₆ à C₆₀, méthoxypolyéthylèneglycol en C₆ à C₆₀ et prényle en C₅ à C₁₂₀; et X est choisi dans le groupe constitué par O, S, N-H, N-méthyle, N-éthyle, N-n-propyle, N-i-propyle et N-R⁶;
leurs stéréoisomères, énantiomères ou diastéréoisomères, leurs sels physiologiquement acceptables ainsi que les mélanges desdits composés pour une utilisation thérapeutique dans l'amélioration des performances mentales.

2. Composé destiné à une utilisation thérapeutique dans l'amélioration des performances mentales, selon la revendication 1, **caractérisé en ce que** R² est un méthoxy.

3. Composé destiné à une application thérapeutique dans l'amélioration des performances mentales, selon l'une des revendications 1 ou 2, **caractérisé en ce que** R³ est H.

4. Composé destiné à une utilisation thérapeutique dans l'amélioration des performances mentales, selon l'une quelconque des revendications 1 à 3, **caractérisé en ce que** la liaison C-C formée par R⁴ et R⁵ est une double liaison.

5. Composé destiné à une utilisation thérapeutique dans l'amélioration des performances mentales, selon la revendication 4, **caractérisé en ce que** la double liaison est une double liaison E.

6. Composé destiné à une utilisation thérapeutique dans l'amélioration des performances mentales, selon l'une quelconque des revendications 1 à 5, **caractérisé en ce que** X est O.

7. Composé destiné à une utilisation thérapeutique dans l'amélioration des performances mentales, selon l'une quelconque des revendications 1 à 6, **caractérisé en ce que** R⁶ est un alkyle en C₈ à C₂₈, de préférence un alkyle en C₁₂ à C₂₄ et de manière particulièrement préférée un alkyle en C₈, un alkyle en C₁₂, un alkyle en C₁₆ ou un alkyle en C₂₀.

8. Composé destiné à une utilisation thérapeutique dans l'amélioration des performances mentales, selon la revendication 1, **caractérisé en ce que** le composé est choisi dans le groupe constitué par (2*E*)-3-(4-hydroxy-3-méthoxyphényl)-prop-2-énoate d'icosyle, (2*E*)-3-(4-hydroxy-3-méthoxyphényl)-prop-2-énoate d'octyle, (2*E*)-3-(4-hydroxy-3-méthoxyphényl)-prop-2-énoate d'hexadécyle, 3-(4-hydroxy-3-méthoxyphényl)-propanoate d'icosyle, (2*E*)-3-(4-hydroxy-3-méthoxyphényl)-prop-2-énoate de dodécyle et (2*E*)-3-(4-hydroxyphényl)-prop-2-énoate d'icosyle.

9. Composé destiné à une utilisation thérapeutique dans l'amélioration des performances mentales, selon l'une quelconque des revendications 1 à 8, dans lequel les performances mentales comprennent les performances de mémoire et les performances d'apprentissage et sont associées à une démence.

10. Composition pharmaceutique destinée à une utilisation thérapeutique dans l'amélioration des performances mentales, comprenant au moins un composé selon l'une quelconque des revendications 1 à 9, ainsi que les sels et stéréoisomères, énantiomères, diastéréoisomères, ou mélanges de ceux-ci, physiologiquement acceptables, et des excipients et additifs pharmaceutiquement acceptables.

11. Composition pharmaceutique destinée à une utilisation thérapeutique dans l'amélioration des performances mentales, selon la revendication 10, en combinaison avec d'autres substances actives choisies dans le groupe constitué par les compléments alimentaires, les améliorants synergiques, les améliorants additifs, les composés pour améliorer l'absorption et le transport et les agents pour réduire le clivage ou la métabolisation, ainsi que leurs mélanges.

12. Composition pharmaceutique destinée à une utilisation thérapeutique dans l'amélioration des performances mentales, selon la revendication 10 ou la revendication 11, **caractérisée en ce que** la composition pharmaceutique est adaptée pour une administration orale, intranasale ou sous-cutanée.

13. Utilisation non thérapeutique d'un composé de formule I tel que représenté dans la revendication 1,
dans laquelle
R¹ est un hydroxy,
R² est choisi dans le groupe constitué de H, méthoxy, éthoxy, n-propoxy et i-propoxy; et
R³ est choisi dans le groupe constitué par H, méthoxy, éthoxy, n-propoxy et i-propoxy;
R⁴ et R⁵ sont chacun indépendamment H ou un R⁴ et un R⁵ forment ensemble une liaison C-C, de telle sorte qu'il existe une liaison simple, double ou triple entre les atomes de C adjacents sur lesquels se trouvent R⁴ et R⁵;
R⁶ est choisi dans le groupe constitué par les groupes alkyle en C₆ à C₆₀ non ramifiés ou ramifiés, alcényle en C₆ à C₆₀ non ramifiés ou ramifiés, hydroxypolyéthylèneglycol en C₆ à C₆₀, méthoxypolyéthylèneglycol en C₆ à C₆₀ et prényle en C₅ à C₁₂₀ ; et
X est choisi dans le groupe constitué par O, S, N-H, N-méthyle, N-éthyle, N-n-propyle, N-i-propyle et N-R⁶;
leurs stéréoisomères, énantiomères ou diastéréoisomères, leurs sels physiologiquement acceptables ainsi que les mélanges desdits composés, dans l'amélioration des performances mentales.
